# EUROPEAN PATENT APPLICATION

(11) **EP 4 768 067 A1**
(43) Date of publication of application: **01.07.2026**
(21) Application number: 25227426.1
(22) Date of filing: 29.12.2025
(51) Int. Cl.: A61M 37/00

(54) **INTEGRATED MICROSTRUCTURE PATCH PROCESSING DEVICE AND METHOD OF USING THE SAME**

(30) Priority: 30.12.2024 US 202463740033 P; 18.11.2025 TW 114144979
(71) Applicant: WCC BIOMEDICAL Co., Ltd., Hsinchu City 300096 (TW)
(72) Inventor: LIU, Ta-Jo, 300096 Hsinchu City (TW); TSENG, Ching-Chen, 300096 Hsinchu City (TW); WU, Yao-Chi, 300096 Hsinchu City (TW); LIU, Chin-Chia, 300096 Hsinchu City (TW); LIEN, Wen-Hsu, 300096 Hsinchu City (TW); LAWBY, Edison, 300096 Hsinchu City (TW); YEH, Hsiu-Feng, 300096 Hsinchu City (TW); CHANG, Chao-Chien, 300096 Hsinchu City (TW); LO, Chi-Fen, 300096 Hsinchu City (TW)
(74) Representative: Marks & Clerk LLP

(57) **Abstract**

An integrated microstructure patch processing device includes a container body unit (1), a support unit (2), a coating device (3), and a processing unit (6). The container body unit (1) includes a shell body (11) that defines an accommodation space (12). The support unit (2) includes a support platform (21) that is disposed in the accommodation space (12), and that has a supporting part (211) for carrying microstructure patches (91). The coating device (3) is disposed in the accommodation space (12) and conducts a coating process which impart a transdermally active substance the microstructure patches. The processing unit (6) is disposed in the accommodation space (12) to conduct a first processing procedure on the microstructure patches (91). A method of processing microstructure patches (91) is included.

## Description

The disclosure relates to a microstructure patch processing device and a method of using the microstructure patch processing device, and more particularly to a device and a method for processing microstructure patches with microneedles for minimally invasive transdermal delivery of drugs or cosmetics.

Microneedles (MNs) are a drug delivery method where medication or other active molecules are absorbed through the skin transdermally and enter the body's circulation via capillaries. This dosage form offers several advantages: high patient compliance, avoidance of the first-pass effect (where drugs are metabolized by the liver before entering circulation), stable and controllable blood concentration levels, and overall high safety. Additionally, microneedles are minimally invasive, painless, safe, and convenient to use. These combined characteristics allow microneedles to be applied across a very wide range of fields, including skin care, drug delivery, vaccine administration, Traditional Chinese Medicine acupuncture, and biosensor technology.

Conventional microneedle manufacturing processes primarily involve steps such as molding, blanking, drying, demolding, and packaging. These methods rely on dispersed, independent equipment for each step, and semifinished products must be exposed to the air during transfer between stations. Additionally, traditional preparation methods involve high-volume batch production, leading to long lead times for new molds and raw material procurement when different product specifications are required.

Consequently, these methods present several drawbacks such as high manufacturing costs, high space requirements for equipment, high risk of contamination, and lack of flexible configuration capabilities for customization.

With the increasing demand for personalized skin care and precision medicine, conventional microneedle preparation methods are no longer able to meet the customized production needs of regional medical institutions or consumers.

To address these limitations, several types of attempts have been made to use 3D printing equipment for microneedle manufacturing. According to existing reports, methods for incorporating drugs or active ingredients into microneedle materials using additive manufacturing technologies include noncontact material deposition, such as Inkjet printing, or photopolymerization-based technologies, including, stereolithography (SLA), digital light processing (DLP), and fused deposition modeling (FDM) technology.

While 3D printing methods offer significant advantages such as lower costs and reduced material waste compared to traditional manufacturing, they face critical limitations that hinder widespread adoption. These methods often struggle to meet the strict requirements for the stability of active pharmaceutical ingredients and the necessary compatibility with various polymers used in the manufacturing process.

Crucially, the mechanical strength, long-term stability, and sterility (bioburden) of microneedles produced via 3D printing often cannot be effectively or consistently validated. This current inability to achieve robust verification makes it challenging to obtain essential medical regulatory certification, which ultimately compromises the safety assurance for both consumers and personalized medical institutions.

Given these persistent challenges, there is an urgent need in the industry for an integrated microneedle carrier manufacturing device. Such a system must combine the benefits of 3D printing, namely low manufacturing cost, minimal space requirements, low contamination risk, and highly flexible configuration capabilities, while also ensuring compatibility with diverse active ingredients and producing verifiable, standardized microneedle specifications to safely meet the growing demand for personalized medicine and advanced skin care.

Therefore, an object of the disclosure is to provide an integrated microstructure patch processing device and a method of using the microstructure patch processing device for processing a microstructure patch that can alleviate at least one of the drawbacks of the prior art.

According to the disclosure, there is provided an integrated microstructure patch processing device according to claim 1.

According to another aspect of the disclosure, there is provided a method of processing a microstructure patch according to claim 22.

Other features and advantages of the disclosure will become apparent in the following detailed description of the embodiment(s) with reference to the accompanying drawings. It is noted that various features may not be drawn to scale.
Figure 1 is a perspective view illustrating a first embodiment of an integrated microstructure patch processing device according to the present disclosure.
Figure 2 is a fragmentary perspective view illustrating the first embodiment.
Figure 3 is a block diagram illustrating the first embodiment.
Figure 4 is a side view illustrating the first embodiment.
Figure 5 is a fragmentary perspective illustrating a coating device conducting a coating process in the first embodiment.
Figure 6 is a top view illustrating the first embodiment.
Figure 7 is a flow diagram illustrating an embodiment of a method of processing a microstructure patch using the integrated microstructure patch processing device.
Figures 8 to 11 are top views illustrating steps in the embodiment of the method.
Figure 12 is a side view illustrating the embodiment of the method.
Figure 13 is a diagram plotting mechanical strength of different microneedles over their displacement.
Figure 14 is a diagram plotting the mechanical strength of different microneedles over compression displacement.
Figure 15 is a fragmentary side view illustrating a second embodiment of the integrated microstructure patch processing device according to the present disclosure.
Figure 16 is a perspective view illustrating a third embodiment of the integrated microstructure patch processing device according to the present disclosure.
Figure 17 is a fragmentary cross-sectional view illustrating the third embodiment.
Figure 18 is a top view illustrating a fourth embodiment of the integrated microstructure patch processing device according to the present disclosure.

Before the disclosure is described in greater detail, it should be noted that where considered appropriate, reference numerals or terminal portions of reference numerals have been repeated among the figures to indicate corresponding or analogous elements, which may optionally have similar characteristics.

It should be noted herein that for clarity of description, spatially relative terms such as "top," "bottom," "upper," "lower," "on," "above," "over," "downwardly," "upwardly" and the like may be used throughout the disclosure while making reference to the features as illustrated in the drawings. The features may be oriented differently (e.g., rotated 90 degrees or at other orientations) and the spatially relative terms used herein may be interpreted accordingly.

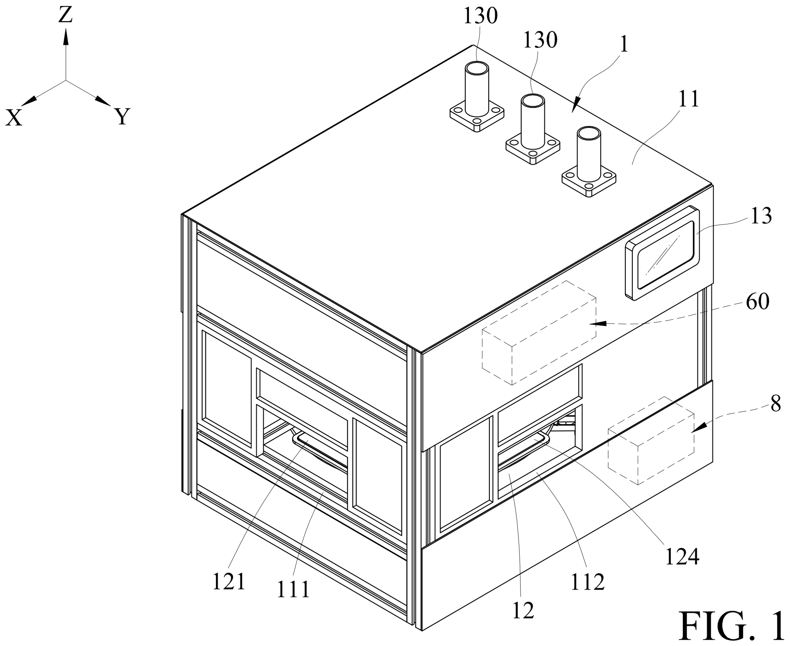

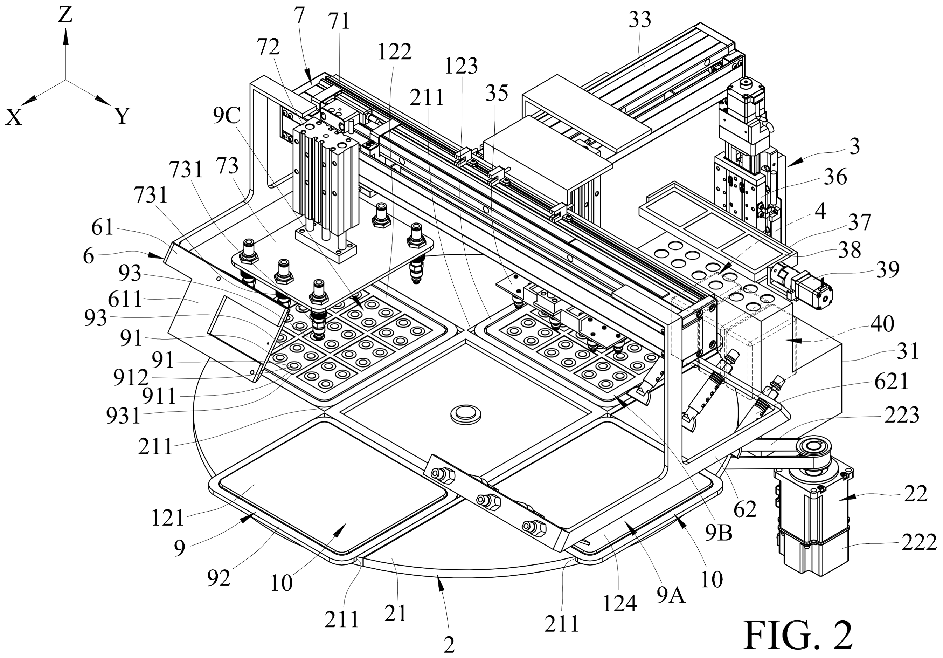

Referring to Figures 1 and 2, a first embodiment of an integrated microstructure patch processing device according to the present disclosure is adapted to apply a transdermally active substance on a plurality of microstructure patches 91. Each of the microstructure patches 91 may have dimensions that are no larger than 5 cm x 5 cm. Each of the microstructure patches 91 has an adhesive layer 911, and a microstructure layer 912 disposed on the adhesive patch layer 911. The microstructure layer 912 may include a plurality of microneedles arranged in an array (not shown in the figures); each of the microneedles may have a length that ranges from 100 µm to 1000 µm. In other embodiments, the microstructure layer may include a plurality of hollow needles, a plurality of micro-pores, a plurality of voids, or other convex or concave structures.

The microstructure layer 912 may be made of a biocompatible material. For example, the microstructure layer 912 may be made of polyether ether ketone (PEEK), polymethyl methacrylate (PMMA), polycarbonate (PC), polylactic acid (PLA), acrylonitrile butadiene styrene (ABS), polypropylene (PP), polyvinylpyrrolidone (PVP), polyvinyl alcohol (PVA), silicone, epoxy resin, or other biocompatible thermoplastic or thermosetting polymer materials of hydrogels. The biocompatible hydrogel may be glycosaminoglycans, polysaccharides, collagen, elastin, fibroin, starch, glucomannan, hyaluronic acid, crosslinked hyaluronic acid, hydrophobically modified hyaluronic acid, or any combination thereof.

The transdermally active substance may include a drug-containing care component or a drug-containing active component. The drug-containing care component may include a drug, a biological preparation, a traditional Chinese medicine, a health food, or a cosmetic. The drug-containing active component may include a water-soluble drug, a fat-soluble drug, a nucleic acid drug, a protein drug, a vaccine, an exosome, a water-soluble traditional Chinese medicine, a water-soluble vitamin, or a natural phenolic antioxidant etc.

In this embodiment, the microstructure layer 912 is a hydrogel. Specifications for a blank microstructure patch (the microstructure patch before it is applied with a transdermally active substance) are shown in the table 1.

| parameters | specification |
|---|---|
| Material | Polyacrylamide-based and chemical cross-linked high-performance hydrogel |
| Viscosity | 150 ± 50 cps at 25 °C |
| PH Value | 4.5~7.0 |
| Expansion Ration | ≧ 200 % |
| Needle length | 900 µm |
| Needles per patch | 279 /patch |
| Microstructure layer area | 1.76 cm² |
| Usage | Depends on the coated transdermally active substance |
| Maximum load | 100 mg/patch |
| Microstructure layer structure | Single layer |
| Mechanical strength | >0.058N |

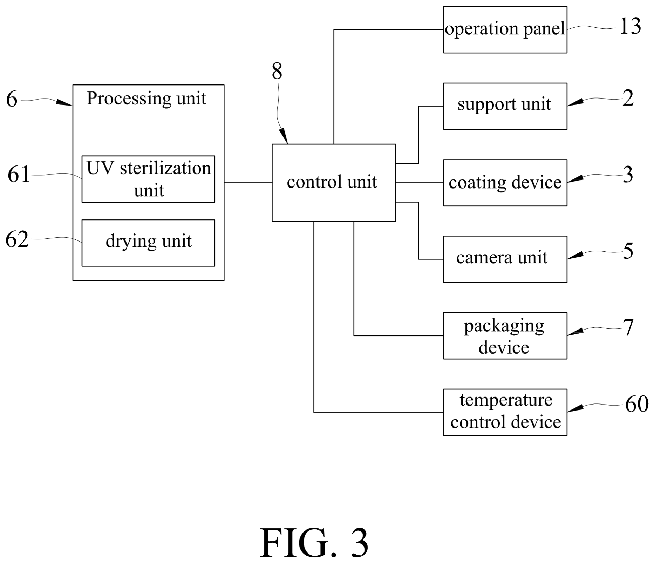

Referring to Figures 1 to 3, the integrated microstructure patch processing device includes a container body unit 1, a support unit 2, a coating device 3, at least one liquid container 4, a recovery container 40, a camera unit 5, a processing unit 6, a temperature control device 60, a packaging device 7, and a control unit 8.

The container body unit 1 includes a shell body 11, an operation panel 13, and a plurality of gas pipes 130.The shell body 11 surrounds and defines an accommodation space 12. The operation panel 13 is disposed on the shell body 11. The gas pipes 130 are mounted to the shell body 11 and fluidly communicate the accommodation space 12 with the external atmosphere. The shell body 11 has an inlet opening 111, and an outlet opening 112 that both spatially communicate the accommodation space 12 with the external atmosphere. The support unit 2, the coating device 3, the processing unit 6, the temperature control device 60, and the packaging device 7 are disposed in the accommodation space 12. More specifically, in this embodiment, the container body unit 1 has a height in an up-down direction (Z) that is 683 mm, a length in a front-rear direction (X) that is perpendicular to the up-down direction (Z) which is 830 mm, and a length in a left-right direction (Y) that is perpendicular to the up-down direction (Z) and the front-rear direction (X) which is 690 mm.

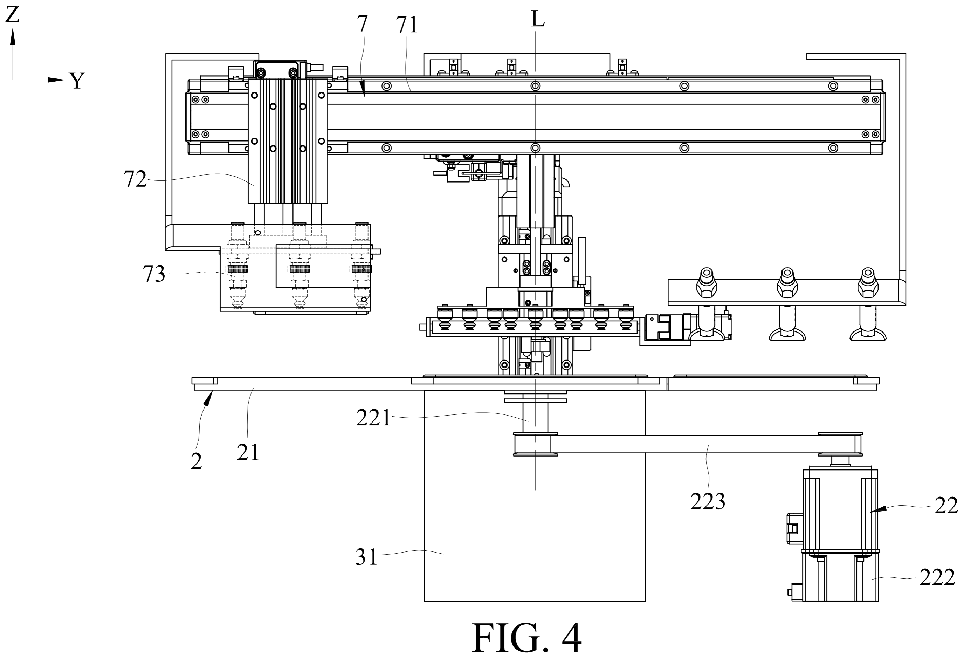

Referring to Figures 1, 2 and 4, the support unit 2 includes a support platform 21 that is disposed in the accommodation space 12, and a moving structure 22. In this embodiment, the support platform 21 is a circular dish and is configured to rotate around a rotation axis (L) along the up-down direction (Z), and the moving structure 22 drives the rotation of the support platform 21.

The support platform 21 has at least one supporting part 211 adapted to carry the microstructure patches 91. In this embodiment, the support platform 21 has four supporting parts 211 that are equiangularly arranged around the rotation axis (L) within the container body unit 1, each supporting part 211 being separated from another one by 90°, and each supporting part 211 is formed on an upper surface of the support platform 21 near the edges thereof. Each of the supporting parts 211 is formed as a recess that is roughly rectangular in shape.

The moving structure 22 has an axel 221, a motor 222, and a transmission belt 223. The axel 221 is immovably mounted to a central portion of a bottom surface of the support platform 21. The transmission belt 223 is connected to the motor 222 and the axel 221.

The accommodation space 12 has an input area 121, a first processing area 122, a coating area 123, and a second processing area 124 that are all located above the support platform 21. In this embodiment, when viewed from above, it is apparent that the input area 121, the first processing area 122, the coating area 123, and the second processing area 124 are arranged in a clockwise order around the rotation axis (L) and are angularly spaced apart by 90° from each other relative to the rotation axis (L).

More specifically, in this embodiment, the input area 121, and the coating area 123 are spaced apart in the front-rear direction (X), the inlet opening 111 of the shell body 11 is adjacent to the input area 121 and is opposite to the coating area 123. The first processing area 122 and the second processing area 124 are spaced apart in the left-right direction (Y). The outlet opening 112 is adjacent to the second processing area 124 and opposite to the first processing area 122.

In an embodiment, the moving structure 22 may be configured to move one of the supporting parts 211 of the support platform 21 between the coating area 123 and the first processing area 122. However, in other embodiments, the moving structure 22 of the support unit 2 may be configured to move each of the supporting parts 211 of the support platform 21 from the input area 121 through the first processing area 122 and the coating area 123 to the second processing area 124. More specifically, the motor 222 of the moving structure 22 is configured to rotate the support platform 21 around the rotation axis (L) via the transmission belt 223 so that each of the supporting part 211 of the support platform 21 may be moved between the input area 121, the first processing area 122, the coating area 123, and the second processing area 124.

The inlet opening 111 is provided for the microstructure patches 91 of the patch unit 9 to enter the input area 121 to reach one of the supporting parts 211 of the support platform 21 of the support unit 2. The microstructure patches 91 of the patch unit 9 may be placed on one of the support parts 211 in the input area 121 through the inlet opening 111 either manually via a worker (not shown in the Figures) or mechanically, for example, via a robot arm (not shown in the Figures). The patch unit 9 may be collected through the outlet opening 112. The outlet opening 112 is provided for the microstructure patches 91 to exit from the second processing area. More specifically, the patch unit 9 may include a tray 92, and nine patch sets 93 arranged in a 3x3 array on the tray 92. In this embodiment, each of the supporting parts 211 is a recessed part of the support platform 21 that is adapted to hold the tray 92 containing the microstructure patches 91. Each of the patch sets 93 has an aluminum film 931, and four microstructure patches 91 arranged in a 2x2 array on the aluminum film 931. The microstructure layer 912 of each of the microstructure patches 91 face upwardly. The tray 92 of the patch unit 9 may have a shape that matches the supporting parts 211 so that when the tray 92 is placed on any one of the supporting parts 211, its position thereof can be fixed, and the microstructure patches 91 can be prevented from shaking or shifting due to the rotation of the support platform 21. In variations of the present embodiment, the microstructure patches 91 may be directly placed on the support platform 21 for processing.

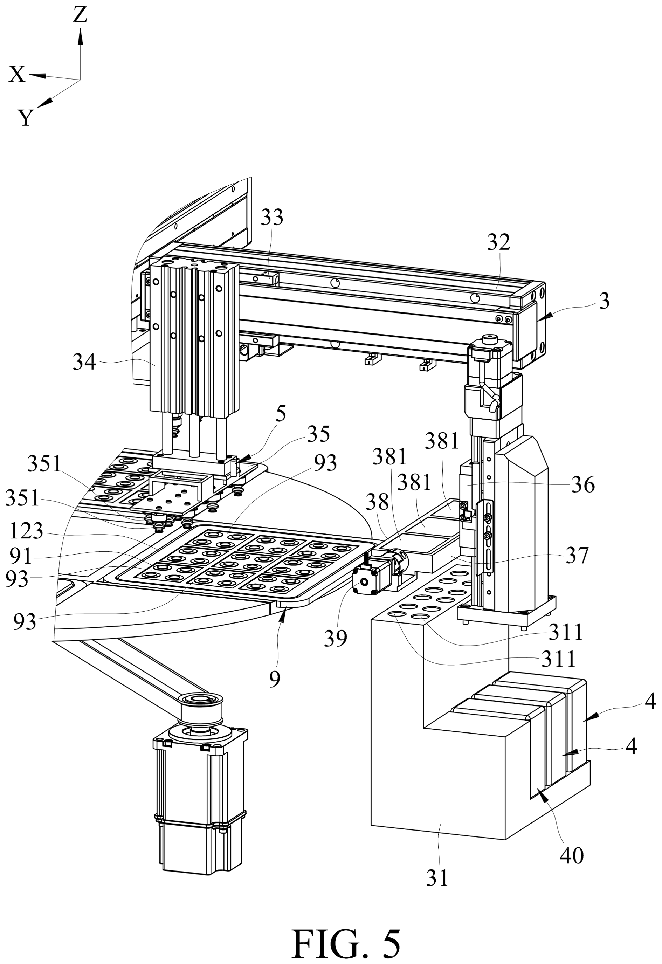

Referring to Figure 5, the coating device 3 is disposed in the accommodation space 12 and is adapted to conduct a coating process which imparts the transdermally active substance on the microstructure layer 912 of each of the microstructure patches 91. For example, when one of the supporting parts 211 carrying the microstructure patches 91 is located in the coating area 123, the coating device 3 may conduct a coating process on the microstructure patches 91. The coating device 3 includes a base seat 31, and a pick-and-place unit 32. The base seat 31 is located proximately to the coating area 123, and is formed with a plurality of receptacles 311. More specifically, in this embodiment, the base seat 31 is formed with 12 receptacles 311 that are adapted to accommodate the transdermally active substance, and are positioned to correspond to the microstructure patches 91 of three of the patch sets 93 on the same column of the 3x3 array.

The pick-and-place unit 32 is adapted to grab the microstructure patches 91 and immerse the microstructure layers 912 of the microstructure patches 91 in the transdermally active substance accommodated in the receptacles 311. The pick-and-place unit 32 includes a horizontal slide structure 33 located above the coating area 123 and extending along the front-rear direction (X), an actuator 34 disposed on the horizontal slide structure 33, a gripping component 35 disposed on the actuator 34, a lifting slide structure 36 disposed beside the seat body 31 and extending along the up-down direction (Z), a frame 37 disposed on the lifting slide structure 36, a holding seat 38 pivotally connected to the frame 37 so as to be able to flip up and down and positioned above the receptacles 311, and a motor 39 disposed on the frame 37 and capable of driving the holding seat 38 to rotate.

The horizontal slide structure 33 is capable of driving the gripping component 35 to move along the front-rear direction (X), and the actuator 34 is capable of driving the gripping component 35 to move along the up-down direction (Z). The gripping component 35 has a plurality of vacuum suction nozzles 351 used for sucking three patch sets 93 in the same column of the 3x3 array on the tray 92. The lifting slide structure 36 is capable of driving the frame 37 to move along the up-down direction (Z). The holding seat 38 has three suction surfaces 381 facing upward. These suction surfaces 381 are used for sucking the three patch sets 93 picked up by the gripping component 35 from the tray 92. In other variations of this embodiment, the combination of the horizontal slide structure 33 and the actuator 34 may alternatively be manifested as a robotic arm or another type of moving device.

In this embodiment, the integrated microstructure patch manufacturing equipment includes two liquid containers 4 that are removably mounted on the base seat 31 of the coating device 3 and fluidly communicating with the coating device 3. The liquid containers 4 may be respectively filled with the transdermally active substance and a cleaning solution. The liquid containers 4 are used to provide the cleaning solution and different transdermally active substances to the receptacles 311. The recovery container 40 is detachably installed on the base seat 31 and can recover the liquid within the receptacles 311. Therefore, when manufacturing microstructure patches 91 with different drug components, the coating device 3 will conduct a cleaning process that supplies the cleaning solution from the liquid containers 4 to the receptacles 311 to dilute and wash out the remaining transdermally active substance in the receptacles 311, and the recovery container 40 recovers the liquid drained from the receptacles 311. Subsequently, the liquid containers 4 will refill the receptacles 311 with the transdermally active substance containing different active ingredients. Specifically, the aforementioned dilution and wash out steps may be repeated multiple times to ensure the thorough cleaning of the receptacles 311, thus enabling the coating device 3 and the liquid containers 4 to accommodate different transdermally active substances containing various active ingredients.

In this embodiment, the coating device 3 applies the transdermally active substance to the microstructure layers 912 of the microstructure patches 91 using a dip coating method. In other variations of this embodiment, the coating device 3 may apply the transdermally active substance to the microstructure layers 912 of the microstructure patches 91 using inkjet printing, nozzle printing, jet drying, spraying, piezoelectric inkjet printing, or a dispensing method.

The camera unit 5 is disposed on the gripping component 35 of the pick-and-place unit 32 and is adapted to capture an image of the microstructure patches 91.

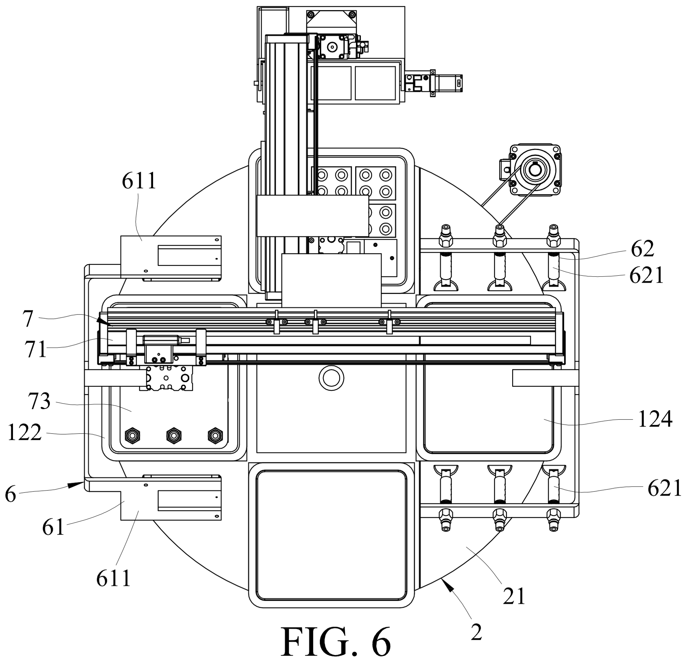

Referring to Figures 2 and 6, the processing unit 6 is disposed in the accommodation space 12 and is adapted to conduct a first processing procedure on the microstructure patches 91. For example, when one of the supporting parts 211 carrying the microstructure patches 91 is located in the first processing area 122, the processing unit 6 may conduct a first processing procedure on the microstructure patches 91. In another example, when one of the supporting parts 211 carrying the microstructure patches 91 are located in the second processing area 124, the processing unit may conduct a second processing procedure on the microstructure patches 91. The processing unit 6 includes a UV sterilization unit 61 that is proximate to the first processing area 122 to sterilize the microstructure patches 91, and a drying unit 62 that is proximate to the second processing area 124 to dry the microstructure patches 91.

The UV sterilization unit 61 has two ultraviolet (UV) light plates 611 located above the first processing area 122. The UV light plates 611 irradiate UV light towards a portion of the support platform 21 that is located in the first processing area 122. In some embodiments, the first processing procedure may be a sterilization process using UV light to irradiate and sterilize the microstructure patches 91 before conducting the coating process on the microstructure patches 91. Specifically, the UV light plates 611 may provide UV light with a wavelength of 185 nm, and the irradiation time for the first processing procedure can be 0 to 6 hours. In other variations of the present embodiment, the UV sterilization unit 61 can also be replaced with other devices to perform sterilization using different methods, such as Gamma ray sterilization or ethylene oxide (EO) sterilization.

In some embodiments, the second processing procedure is a drying process for drying the transdermally active substance on the microstructure layer 912 of each of the microstructure patches 91 after the coating process has been conducted on the microstructure patches 91. In this embodiment, the drying unit 62 has six nozzles 621 located above the second processing area 124. The nozzles 621 are used to output gas towards a portion of the support platform 21 that is located in the second processing area 124. The nozzles 621 are fluidly communicated with a gas source (not shown in the figures) through at least one of the gas pipes 130, and this gas source may be filtered or heated dry air. For example, the drying unit 62 may perform the second processing procedure by outputting gas to the coated microstructure patches 91 in the second processing area 124 to dry the transdermally active substance on the microstructure layers 912.

In other variations of the present embodiment, the drying unit 62 can also be a device that performs drying using other methods such as convection drying, radiation drying, infrared drying, microwave drying, vacuum drying, or freeze-drying. The drying unit 62 may be a fan device, an electric heating tube, or a freeze dryer. In one embodiment, the drying unit 62 may be a fan device (not shown in the figure) arranged above the box unit 1. The fan device may include a fan and a filter. The rotation speed of the fan may be 3000 rpm, and the filter can be a high-efficiency particulate air (HEPA) filter or an air filter membrane.

It should be noted that the first processing procedure described in the present embodiment is not limited to sterilization or drying. In other variations of the present embodiment, the processing unit 6 may only include one of the UV sterilization unit 61, the drying unit 62, or other devices to process the microstructure patches 91.

In one embodiment, the processing unit 6 may only include the UV sterilization unit 61 for performing the first processing procedure to sterilize the microstructure patches 91 before the coating device 3 performs the coating process, after which the microstructure patches 91 may undergo natural drying upon completion of the coating process.

In another embodiment, the processing unit 6 may solely include the drying unit 62 for performing the first processing procedure in order to dry the microstructure patches 91 after the coating device 3 performs the coating process.

The temperature control device 60 can control the temperature of the accommodation space 12. Specifically, the temperature control device 60 may be an air conditioning unit that is able to maintain the accommodation space 12 at a set temperature ranging from -80 to 80°C. The set temperature can also be - 80°C, -60°C, -40°C, -20°C, 0°C, 20°C, 40°C, 60°C, or 80°C.

Referring back to Figs. 2, 4 and 6, the packaging device 7 is disposed in the accommodation space 12 and is configured to conduct a packaging procedure that grabs a packaging member 10 (see Fig. 2) and that covers the microstructure patches 91 with the packaging member 10. In particular, the packaging device 7 conducts the packaging procedure that picks up the packaging member 10 arriving at the first processing area 122 and covers the microstructure patches 91 arriving at the second processing area 124.

The packaging device 7 includes a horizontal slide structure 71 located above the support platform 21 and extending between the first processing area 122 and the second processing area 124, an actuator 72 disposed on the horizontal slide structure 71, and a suction unit 73 mounted on the actuator 72.

In this embodiment, the first and second processing areas 122, 124 are aligned along the left-right direction Y (i.e., the y-axis direction perpendicular to the rotational axis L). The horizontal slide structure 71 can drive the actuator 72 and the suction unit 73 to move between the first processing area 122 and the second processing area 124, and the actuator 72 can drive the suction unit 73 to move along the up-down direction (Z). The suction unit 73 has a plurality of vacuum suction nozzles 731 for suctioning a packaging member 10 and is movable between the first and second processing areas 122, 124 to pick up the packaging member 10 in the first processing area 122 and to transfer the packaging member 10 to the second processing area 124, the suction unit 73 is operable to be placed on and to cover the microstructure patches 91 arriving at the second processing area 124. The packaging member 10 is made of transparent PET material and is used to cover the patch unit 9, and can be hot-pressed with the aluminum films 931 (see Fig. 2) to hermetically seal the microstructure patches 91. Specifically, the hot pressing temperature may range from 30°C to 150°C, and the maximum pressure is 80 kgf.

In other variations of the present embodiment, the aluminum films 931 can also be a plurality of blister packs (not shown in the figure) that can be interlocked with the packaging member 10 to form a blister pack structure that can be sealed together using upper and lower interlocking elements. During sealing, heating is not required; the blister packs and the packaging member 10 can be sealed just by applying pressure to the same.

At least one of the gas pipes 130 can be connected to a vacuum pump to provide vacuum to the vacuum suction nozzles 351 of the gripping component 35, the suction surfaces 381 of the holding base 38, and the vacuum suction nozzles 731 of the suction unit 73.

Referring to Figures 1 to 3, the control unit 8 is disposed in the accommodation space 12 and electrically connected to the operation panel 13, the support unit 2, the coating device 3, the camera unit 5, the processing unit 6, the temperature control device 60, and the packaging device 7.

The control unit 8 is configured to, upon receiving a signal, control the operations of at least one of the moving structure 22 of the support unit 2, the coating device 3, the camera unit 5, the processing unit 6, the temperature monitoring device 60, and the packaging device 7 to perform an automated processing procedure or a manual processing procedure. Specifically, a user can set up the aforementioned devices for an automated processing procedure, or manually perform individual operating procedures, via operating the operation panel 13. In other variations of the present embodiment, the control unit 8 can also receive the control command in other ways, for example, by utilizing wireless transmission to receive the control command from a mobile device, or by receiving the control command via a computer host. The control unit 8 may be configured to identify a positioning of the microstructure patches 91 or the microstructure layer 912 of each of the microstructure patches 91 from an image captured by the camera unit 5, and may be configured to control the coating device 3 to conduct the coating process which imparts the transdermally active substance on the microstructure layer 912 of each of the microstructure patches 91. The control unit 8 can use Al image recognition to identify the positions of the microstructure patches 91 and the microstructure layers 912 based on an image of the microstructure patches 91 captured by the camera unit 5, and control the coating device 3 to perform a precision coating process.

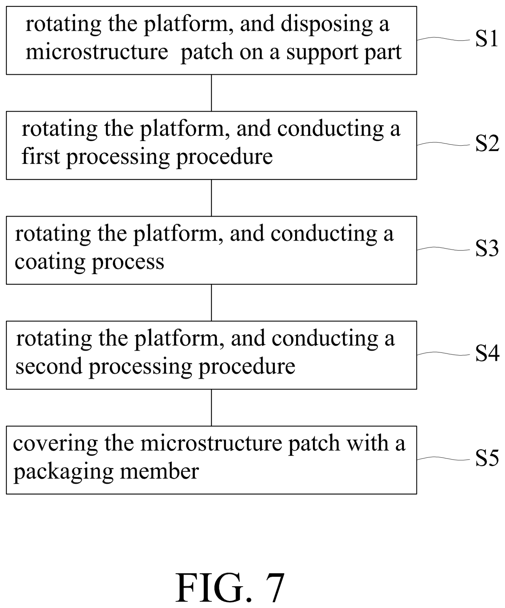

Referring to Figure 7, an embodiment of a method of processing microstructure patches 91 using an integrated microstructure patch processing device includes the steps (S0) (S1), (S2), (S3), (S4), and (S5). In step (S0), a microstructure patch processing device is provided, which is similar to the integrated microstructure patch processing device as described previously. It is noted that in this embodiment of the method, the support platform 21 of the support unit 2 has four support parts 211.

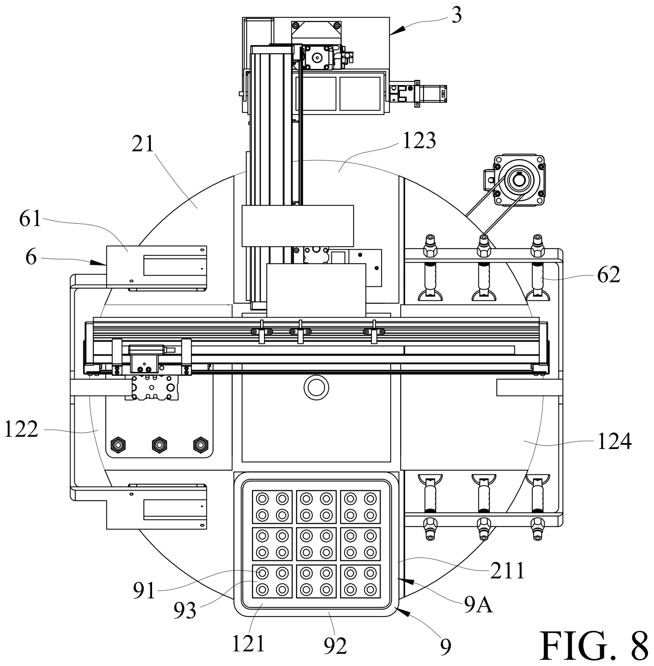

Referring to Figures 1 and 8, in the step (S1) the support platform 21 is rotated to move one of the four support parts 211 to the input area 121 of the accommodation space 12. Next, via a robotic arm or a user manually disposes a patch unit 9 including a plurality of microstructure patches 91 on the support part 211 located in the input area 121 via the inlet opening 111. At this state, the microstructure patches 91 are free of a transdermally active substance. As best illustrated in Figure 2, each of the microstructure patches 91 has an adhesive patch layer 911 and a microstructure layer 912 that is disposed on the adhesive patch layer 911.

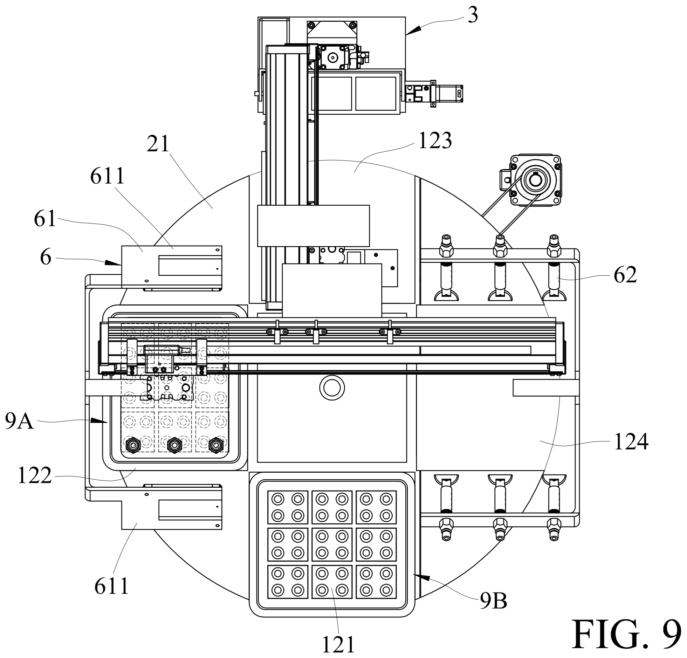

Referring to Figure 9, in the step (S2), the support platform 21 is rotated to move the support part 211 initially located at the input area 121 to the first processing area 122 of the accommodation space 12. Next, another patch unit 9 is disposed on another one of the support parts 211 arriving at the input area 121. For ease of explanation, the first patch unit 9 disposed on the support part 211 is henceforth referred to as the patch unit 9A and the another patch unit 9 next disposed on the support part 211 located in the input area 121 is henceforth referred to as the patch unit 9B. Next, a first processing procedure is conducted by the processing unit 6 in which a UV light is irradiated on the patch unit 9A, thereby sterilizing the patch unit 9A.

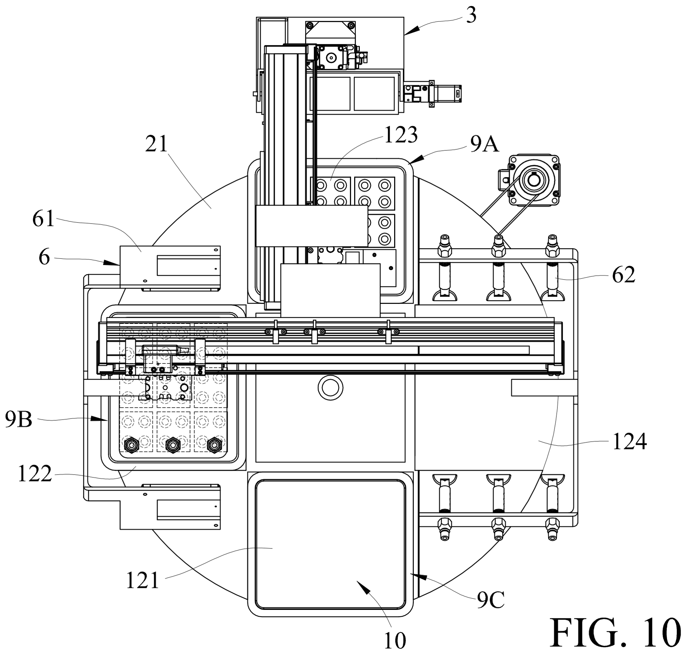

Referring to Figures 5 to 10, in the step (S3), the support platform 21 is rotated again to move the support part 211 with the patch unit 9A to the coating area 123 of the accommodation space 12, and the patch unit 9B is moved to the first processing area 122. A new patch unit 9C is disposed in the support part 211 arriving the input area 121. The patch unit 9C is covered with a packaging member 10. Next, a coating process is conducted by the coating device 3 in which a transdermally active substance is imparted on the patch unit 9A. More specifically, a camera unit 5 that is disposed on the coating device 3 captures an image of the microstructure layer 912 of the patch unit 9A and the control unit 8 which is electrically connected to the camera unit 5 and the coating device 3, identifies a positioning of the microstructure layer 912 of the patch unit 9A and controls the coating device 3 to impart the transdermally active substance on the microstructure layer 912 of the patch unit 9A. In particular, the control unit 8, based on the images of the first patch unit 9A captured by the camera unit 5, controls the horizontal slide structure 33 and the actuator 34 to make the gripping component 35 pick up three of the patch sets 93 arranged in the left-right direction Y (one column in a 3x3 array) on the tray 92 and place them on the suction surfaces 381 of the holding seat 38. The suction surfaces 381 of the holding base 38 suck the microstructure patches 91. Next, the motor 39 drives the holding seat 38 to flip over so that the microstructure layers 912 of the microstructure patches 91 face downward. The lifting slide structure 36 drives the frame 37 and the holding seat 38 downward, immersing the microstructure layers 912 in the transdermally active substance within the receptacles 311. Then, the holding seat 38 moves up and flips over, and the gripping component 35 places the microstructure patches 91, which have been immersed in the transdermally active substance, back onto the tray 92. The same steps are then followed to complete the immersion of the microstructure patches 91 for the remaining six patch sets 93 on the tray 92. Similarly, the UV sterilization unit 61 simultaneously performs the first processing procedure by sterilizing the second patch unit 9B.

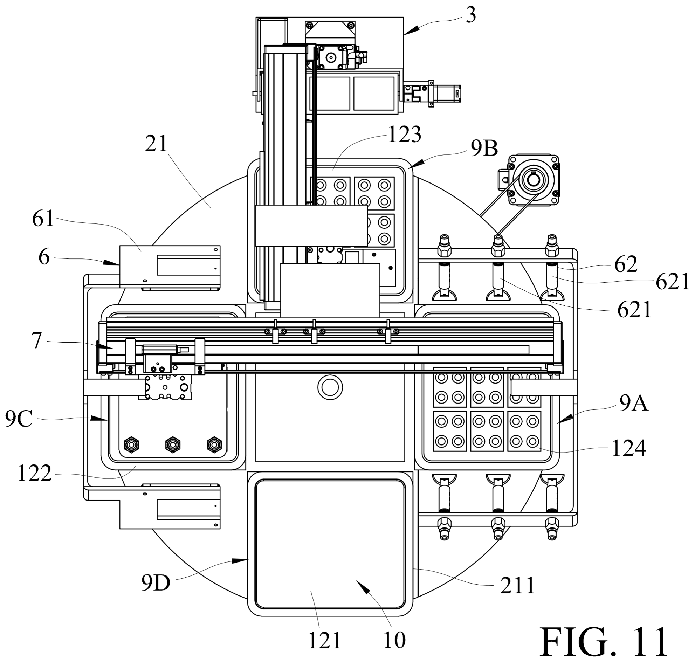

Referring to Figure 11, in the step (S4) of the method, after the patch unit 9A is moved to the second processing area 124 by rotating the support platform, a new patch unit 9D is disposed on the support part 211 that is located in the input area 121. The new patch 9D is covered by the packaging member 10. The second processing procedure is conducted by the drying unit 62 which conducts air drying to dry the patch unit 9A. In particular, the nozzles 621 of the drying unit 62 of the processing unit 6 outputs a gas towards a portion of the support platform 21 that is located in the second processing area 124, and dries transdermally active substance on the microstructure layer 912 of the patch unit 9A. At the same time, the coating device 3 conducts the coating process on the patch unit 9B, and the processing unit 6 conducts the first processing procedure on the patch unit 9C.

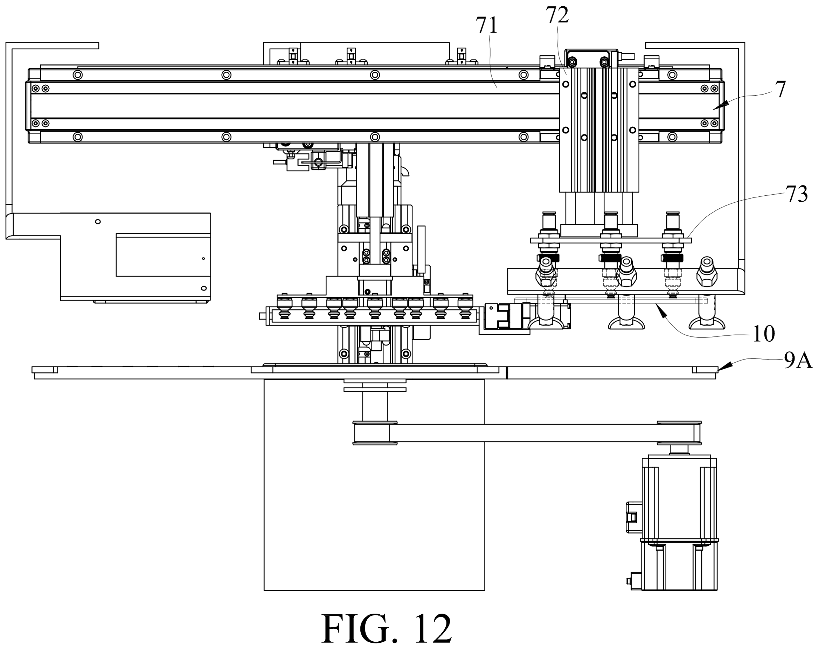

Referring to Figures 1, 11 and 12, in the step (S5), after performing the step of rotating the platform and conducting a second processing procedure, the packaging device 7 covers the patch unit 9A with a packaging member 10. More specifically, the suction unit 73 of the packaging device 7 sucks the packaging member 10 covering the patch 9C and places it over the patch unit 9A to cover the patch unit 9A. This avoids the patch unit 9A from being contaminated after drying. Next, the patch unit 9A is retrieved from the outlet opening 112 of the shell body 11. At this stage, the patch unit 9A has been hot pressed with the packaging member 10. It is noted that the patch sets 93 have the aluminum film 931 which is conducive for hot pressing with the packaging member 10. In this way the sanitation of the microstructure patches 91 of the patch unit 9 may be maintained after sterilization.

It is noted that the method of making microstructure patches may produce one tray 92 of microstructure patches 91 per hour which is 36 microstructure patches 91. In other embodiments of the disclosure the size of the tray 92 and the number and positioning of microstructure patches 91 in each tray 92 may be varied and the amount produced per hour may be different.

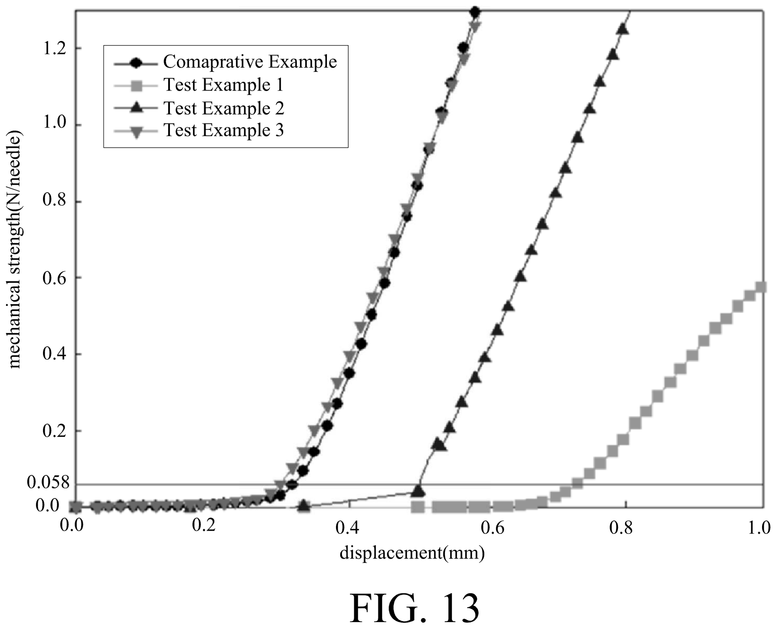

Referring to Figure 13, which shows various mechanical strengths of microstructure patches of Comparative Example, Test Example 1, Test Example 2, and Test Example 3 after experiencing different drying times. Here, the horizontal axis represents microneedle compression displacement, and the vertical axis represents mechanical strength per unit microneedle.

The strength of the microneedles was tested by placing the microstructure layer 912 on a universal testing machine (instrument model 3343, purchased from INSTRON), setting the displacement to 10 millimeters, and conducting a compression test at a speed of 66 mm/min, while collecting 500 compression stress values per second. The mechanical strength of the needle body is represented by the stress measured when the microneedle height is compressed to approximately half (i.e., when the needle body height is about 150 µm). Generally, the mechanical strength measured when the microneedle height is compressed to about half is greater than 0.058 Newtons (N). At this point of compression the microneedle is considered capable of penetrating the human epidermis without the needle body bending or breaking.

The microstructure layers 912 of Comparative Example and Test Examples 1 to 3 are microneedles that include a hydrogel material. Comparative Example is a microstructure patch 91 that was not sprayed with a dye. Test Examples 1 to 3 are microstructure patches 91 coated with a blue dye using the processing method of the present invention, with the difference being that Test Example 1 was dried at a temperature of 30°C for 15 minutes after coating with the dye, Test Example 2 was dried at a temperature of 30°C for 60 minutes after coating with the dye, and Test Example 3 was dried at a temperature of 30°C for 75 minutes after coating with the dye.

The results show that the microneedles dried at 30°C for 75 minutes using the processing method for a microstructure patch of the present invention have the same mechanical strength as the uncoated microneedles. Furthermore, the microneedles in all the Test Examples 1 to 3 and the Comparative Example possess the necessary mechanical strength to pierce human epidermis (such as the scalp) without bending or breaking (i.e., equal to 0.058 N/needle).

When astaxanthin is the transdermally active substance, astaxanthin microneedles can be prepared using the present invention's method for a microstructure patch.

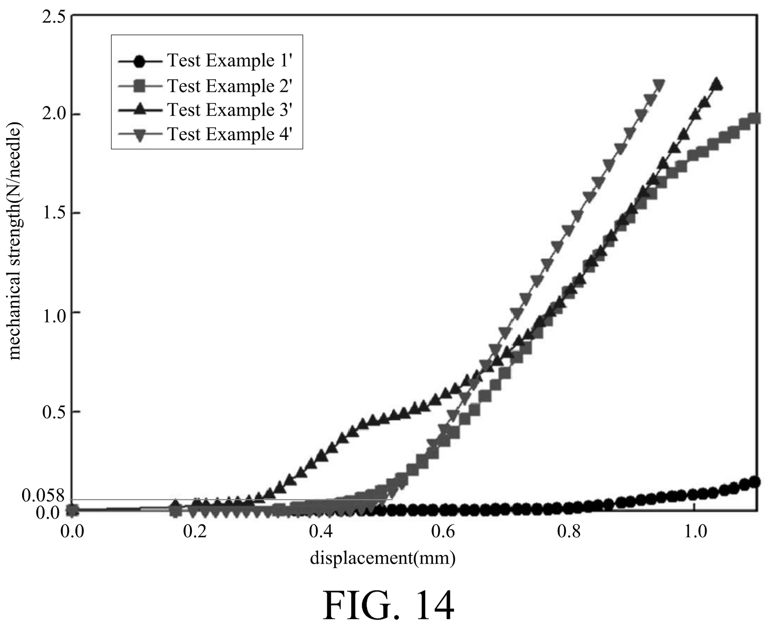

Figure 14 shows different mechanical strengths of microstructure patches 91 for Test Examples 1' through 4', prepared by this method. In Test Example 1', astaxanthin constitutes 100 wt% of the transdermally active substance. In Test Examples 2' to 4', the transdermally active substance comprises astaxanthin and phosphate buffered saline (PBS) buffer solution, with astaxanthin-to-buffer ratios of 3:1, 2:1, and 1:1, respectively

The results show that the astaxanthin microneedles possess the necessary mechanical strength to pierce human epidermis (such as the scalp) without bending or breaking (i.e., equal to 0.058 N/needle).

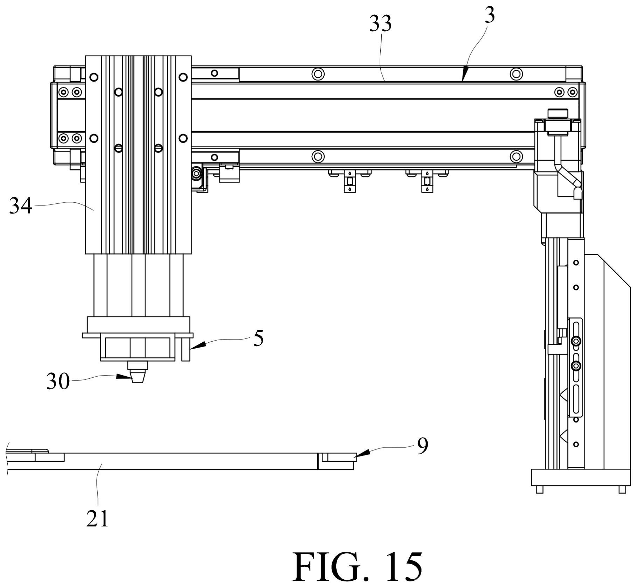

Referring to Figure 15, which shows a second embodiment of the integrated microstructure patch processing device of the present invention, the coating device 3 is a dispensing device that performs the coating process via a dispensing procedure.

Referring to Figure 5 and Figure 15, specifically, the coating device 3 includes the horizontal sliding structure 33, the actuator 34, and a dispenser unit 30 disposed movably within the accommodation space 12. Furthermore, the dispenser unit 30 is positioned on the actuator 34 and connected to the liquid container 4. Similar to the first embodiment, the control unit 8 can, based on an image of the microstructure patches 91 captured by the camera unit 5, control the horizontal sliding structure 33 and the actuator 34 to drive the dispenser unit 30 to move, so that the dispenser unit 30 outputs the transdermally active substance stored in the liquid container 4 on the microstructure layer 912 of each of the microstructure patches 91. When changing the transdermally active substance to be outputted, the dispenser unit 30 can also output the cleaning solution to clean and washout remaining transdermally active substance in the coating device 3. Specifically, the dispenser unit 30 is a dispenser nozzle, using a non-contacting and spraying type of method for dispensing. In other variations of this embodiment, the dispenser unit 30 can also be a dispensing head, with specifications organized as shown in Table 2.

**Table 2**

| Parameter | Specification |
|---|---|
| Model | TS5540 |
| Length | 4.2" (107 mm) |
| Diameter | 1.12" (28 mm) |
| Weight | 0.74lb (336 g) |
| Fluid contact parts | 303 SS , Teflon^{®} Delrin^{®} |
| Viscosity range | 1 to 5k cps |
| Max fluid pressure | 100 psi(6.9 bar) |
| Operating pressure | Minimum: 70 psi (4.8 bar) |
| Connections | Inlet : 1/8" NPT , Outlet : Nozzle tube |
| Nozzle mount size | ¼"-20UNC-2B |

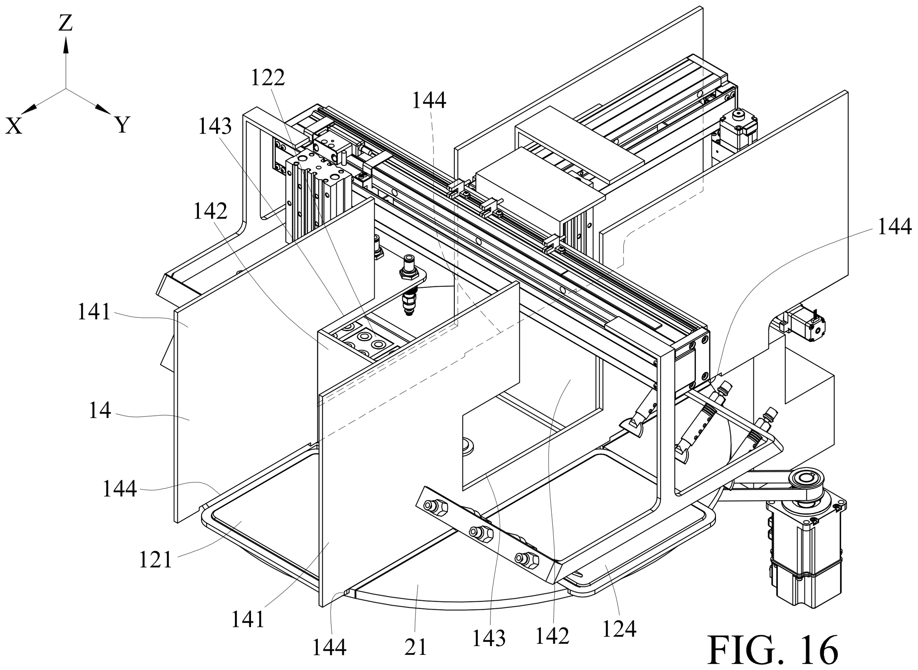

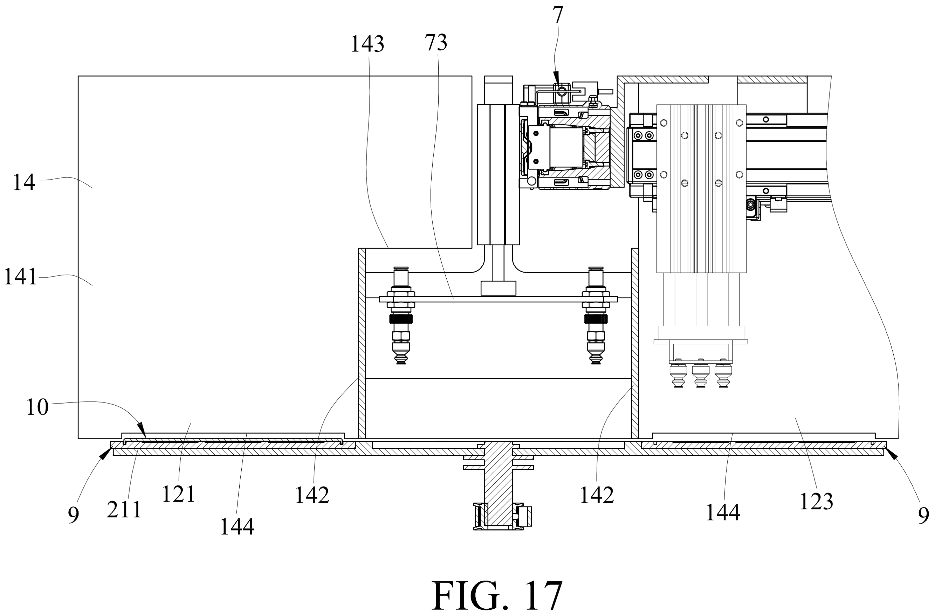

Referring to Figure 16 and Figure 17, a third embodiment of the integrated microstructure patch processing device of the present disclosure is shown. The third embodiment is similar to the first embodiment, with the difference being that the container body unit 1 further includes a partition unit 14 that is fixed to the shell body 11 and located within the accommodation space 12, and that separates the first processing area, the coating area 123, and the second processing area 124 from each other. More specifically, the partition unit 14 is interposed between the coating area 123 and the first processing area 122 and provides shielding between the coating area 123 and the first processing area 122, between the coating area 123 and the second processing area 124, and between the second processing area 124 and the input area 121. In particular, the partition unit 14 has two first partition plates 141 extending along the front-rear direction (X) and spaced apart in the left-right direction (Y). More specifically, the two first partition plates 141 are located on two opposite sides (relative to the left-right direction (Y)) of the input area 121 and the coating area 123. Moreover, it should be noted that the partition unit 14 further has two second partition plates 142 that are interconnecting between the first partition plates 141 and that are spaced apart in the front-rear direction (X). Furthermore, the support platform 21 is rotatable relative to the partition unit 14.

A notch 143 extends through a middle part of each of the first partition plates 141 along the left-right direction (Y). The notches 143 are located between the first processing area 122 and the second processing area 124, and are used for the suction unit 73 of the packaging device 7 as a passage way to move through. The partition unit 14 is formed with a channel 144 that interconnects the coating area 123 and the first processing area 122 and is adapted to allow the microstructure patches 91 to pass through. More specifically, in this embodiment, two channels 144 are formed at the bottom of each first partition plate 141 and are respectively adjacent to the input area 121 and the coating area 123. The channels 144 of the two first partition plates 141 are located on the moving path of the supporting parts 211 to allow the patch unit 9 or the patch unit 9 covered with the packaging member 10 on the supporting part 211 to pass through. Specifically, one of the channels 144 is located between the input area 121 and the first processing area 122, another one of the channels 144 is located between the first processing area 122 and the coating area 123, still another one of the channels 144 is located between the coating area 123 and the second processing area 124, and still another one of the channels 144 is located between the second processing area 124 and the input area 121.

The two second partition plates 142 connecting between the two first partition plates are respectively adjacent to front ends and rear ends of the notches 143 along the front-to-rear direction (X). More specifically, one of the second partition plates 142 and the two first partition plates 141 generally form a U-shape surrounding the input area 121 when viewed from above, and the other one of the second partition plates 142 and the two first partition plates 141 generally form a U-shape surrounding the coating area 123 when viewed from above.

Therefore, when preparing the microstructure patches 91, the partition unit 14 can prevent cross-contamination between the input area 121, the first processing area 122, the coating area 123, and the second processing area 124. Specifically, it prevents the transdermally active substance from being accidentally sprayed onto the first processing area 122, the input area 121, or the second processing area 124 during the coating process which occurs in the coating area 123. It also prevents the transdermally active substance on the microstructure patches 91 from diffusing into the first processing area 122, the input area 121, or the coating area 123 during the drying process which occurs in the second processing area 124.

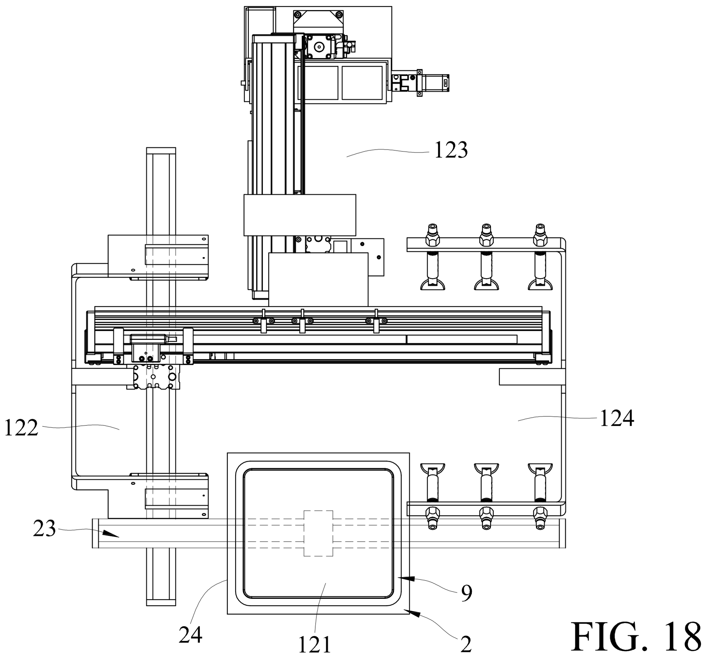

Referring to Figure 18, which shows a fourth embodiment of the integrated microstructure patch processing device according to the present disclosure, the moving structure 22 of the support unit 2 includes an x-y axis slide structure 23 and a carrier 24 disposed on the x-y axis slide structure 23. The carrier 24 is used to carry a patch unit 9. Specifically, the x-y axis slide structure 23 is composed of two 60 cm long slide rails intersecting each other perpendicularly. The x-y axis slide structure 23 can drive the carrier 24 and the patch unit 9 to move between the input area 121, the first processing area 122, the input area 121, and the second processing area 124.

In summary of the above, the integrated microstructure patch processing device of the present disclosure has the following advantages and provides the following effects.

I. The support unit 2, the coating device 3, and the processing unit 6 are installed within the accommodation space 12 defined by the shell body 11 of the container body unit 1, thereby constituting the main structural features of the integrated microstructure patch processing device. This offers the advantage of low space requirements, making it suitable for small-scale personal or home use, as well as for large-scale industrial manufacturing.

II. The support platform 21 drives the microstructure patches 91 to move between the coating area 123, the first processing area 122, and the second processing area 124 by means of rotation. This allows the integrated microstructure patch processing device to have a simple moving mechanism and have the benefit of low manufacturing costs. This also effectively utilizes the accommodation space 12 to miniaturize the overall size of the integrated microstructure patch processing device.

III. By means of the removable liquid container 4 and by outputting the cleaning solution to clean the internal pipelines of the coating device 3, it is convenient to switch out or replace different types of pharmaceutical and cosmetic ingredients.

IV. By using the UV sterilization unit 61 to irradiate the microstructure layers 912 with ultraviolet light for sterilization before coating, the bacterial count on the microstructure layers 912 can be effectively reduced, ensuring the hygiene and safety of the user.

V. By using the drying unit 62 to dry the microneedles on the microstructure layers 912 after coating, it is possible to prevent the transdermally active substance from dispersing and maintain the mechanical strength of the microneedles. This can recover the mechanical strength of the microneedles before conducting the coating, which can make them suitable for skin penetration and ensures reusability.

VI. The partition unit 14 can prevent cross-contamination between the input area 121, the first processing area 122, the coating area 123, and the second processing area 124, thereby maintaining the cleanliness within the accommodation space 12.

VII. In the packaging step of the microstructure patch manufacturing method, covering the dried microstructure patches 91 with the packaging member 10 prevents the microstructure patches 91 from being contaminated by micro-dust particles or volatile gases, which maximizing their cleanliness and provides superior sanitation.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

## Claims

1. An integrated microstructure patch processing device adapted to apply a transdermally active substance on a plurality of microstructure patches (91), each of the microstructure patches (91) having an adhesive patch layer (911), and a microstructure layer (912) disposed on the adhesive patch layer (911), said integrated microstructure patch processing device comprising:
a container body unit (1) including a shell body (11) that surrounds and defines an accommodation space (12);
a support unit (2) including a support platform (21) that is disposed in said accommodation space (12), and that has at least one supporting part (211) adapted to carry the microstructure patches (91);
a coating device (3) disposed in said accommodation space (12), and adapted to conduct a coating process which imparts the transdermally active substance on the microstructure layer (912) of each of the microstructure patches (91); and
a processing unit (6) disposed in said accommodation space (12) and adapted to conduct a first processing procedure on the microstructure patches (91).

2. The integrated microstructure patch processing device as claimed in claim 1, wherein:
said accommodation space (12) has a coating area (123), and a first processing area (122);
said support unit (2) further including a moving structure (22) that is configured to move said at least one supporting part (211) of said support platform (21) between said coating area (123) and said first processing area (122);
when said at least one supporting part (211) carrying the microstructure patches (91) is located in the coating area (123), the coating device (3) conducts a coating process on the microstructure patches (91); and
when said at least one supporting part (211) carrying the microstructure patches (91) is located in said first processing area (122) the processing unit (6) conducts the first processing procedure on the microstructure patches (91).

3. The integrated microstructure patch processing device as claimed in any one of claims 1 and 2, wherein said container body unit (1) further includes a partition unit (14) that is interposed between said coating area (123) and said first processing area (122).

4. The integrated microstructure patch processing device as claimed in claim 3, wherein said partition unit (14) is formed with a channel (144) that interconnects said coating area (123) and said first processing area (122) and that is adapted to allow the microstructure patches (91) to pass through.

5. The integrated microstructure patch processing device as claimed in any one of claims 2 to 4, wherein:
said accommodation space (12) further has a second processing area (124);
said moving structure (22) of said support unit (2) is configured to move said at least one supporting part (211) of said support platform (21) from said first processing area (122) and said coating area (123) to said second processing area (124); and
when said at least one supporting part (211) carrying the microstructure patches (91) are located in the second processing area (124), the processing unit (6) conducts a second processing procedure on the microstructure patches (91).

6. The integrated microstructure patch processing device as claimed in any one of claims 2 to 5, wherein:
the first processing procedure is a sterilization process that is conducted before conducting the coating process to sterilize the microstructure patches (91); and
said processing unit (6) includes a UV sterilization unit (61) that is used to perform the sterilization process for sterilization of the microstructure patches (91).

7. The integrated microstructure patch processing device as claimed in any one of claims 5 and 6, wherein:
the second processing procedure is a drying process for drying the transdermally active substance on the microstructure layer (912) of each of the microstructure patches (91) after the coating process has been conducted on the microstructure patches (91); and
said processing unit (6) further includes a drying unit (62) that conducts air drying to dry the microstructure patches (91) and that is used to perform the drying process.

8. The integrated microstructure patch processing device as claimed in any one of claims 2 and 5 to 7, wherein said container body unit (1) further includes a partition unit (14) that separates said first processing area (122) said coating area (123), and said second processing area (124) from each other.

9. The integrated microstructure patch processing device as claimed in any one of claims 2 to 8, wherein:
said coating area (123) and said first processing area (122) are angularly spaced apart from each other relative to a rotation axis (L); and
said moving structure (22) of said support unit (2) is configured to rotate said support platform (21) around the rotation axis (L) to move said at least one supporting part (211) between said coating area (123) and said first processing area (122).

10. The integrated microstructure patch processing device as claimed in any one of claims 2 to 9, wherein said moving structure (22) includes an x-y axis slide structure (23).

11. The integrated microstructure patch processing device as claimed in any one of claims 1 to 10, wherein said at least one supporting part (211) is a recessed part of said support platform (21) that is adapted to hold a tray (92) containing the microstructure patches (91).

12. The integrated microstructure patch processing device as claimed in any one of claims 1 to 11, further comprising
a camera unit (5) that is disposed on said coating device (3), and that is adapted to capture an image of the microstructure patches (91), and
a control unit (8) that is electrically connected to said camera unit (5) and said coating device (3), that is configured to identify a positioning of the microstructure patches (91) from the image captured by the camera unit (5), and that is configured to control the coating device (3) to conduct the coating process which impart the transdermally active substance on the microstructure layer (912) of each of the microstructure patches (91).

13. The integrated microstructure patch processing device as claimed in any one of claims 1 to 11 further comprising a control unit (8) electrically connected to said support unit (2), said coating device (3), and said processing unit (6), and configured to, upon receiving a signal, control at least one of said support unit (2), said coating device (3), and said processing unit (6) to perform an automatic processing procedure or a manual processing procedure.

14. The integrated microstructure patch processing device as claimed in any one of claims 1 to 13, wherein said coating device (3) includes a dispenser unit (30) that is disposed and movable within said accommodation space (12), and that is adapted to dispense the transdermally active substance on the microstructure layer (912) of each of the microstructure patches (91).

15. The integrated microstructure patch processing device as claimed in any one of claims 1 to 14, wherein said coating device (3) includes
a base seat (31) that is formed with a plurality of receptacles (311) adapted to accommodate the transdermally active substance, and a pick-and-place unit (32) that is adapted to grab the microstructure patches (91) and immerse the microstructure layers (912) of the microstructure patches (91) in the transdermally active substance accommodated in the receptacles (311).

16. The integrated microstructure patch processing device as claimed in any one of claims 1 to 15 further comprising a liquid container (4) that is removably mounted to and fluidly communicating with said coating device (3), and that is adapted to hold the transdermally active substance.

17. The integrated microstructure patch processing device as claimed in any one of claims 1 to 15, wherein:
said integrated microstructure patch processing device further comprises a liquid container (4) that is fluidly communicated with said coating device (3), and that is adapted to hold the transdermally active substance or a cleaning solution; and
said coating device (3) is configured to conduct a cleaning process that outputs the cleaning solution to washout remaining transdermally active substance in said coating device (3).

18. The integrated microstructure patch processing device as claimed in any one of claims 1 to 17, further comprising a packaging device (7) disposed in said accommodation space (12) and being configured to conduct a packaging procedure that picks up a packaging member (10) and that covers the microstructure patches (91) with said packaging member (10).

19. The integrated microstructure patch processing device as claimed in claim 1, wherein:
said accommodation space (12) has an input area (121), a first processing area (122), a coating area (123), and a second processing area (124) all of which are angularly spaced apart around a rotation axis (L) in said accommodation space,
said support unit (2) further has a moving structure (22) to move said at least one supporting part (211) of said support platform (21) from said input area through said first processing area (122) and said coating area (123) to said second processing area (124);
said shell body (11) has an inlet opening (111) for the microstructure patch (91) to enter said input area (121) to reach said at least one supporting part (211), and an outlet opening (112) for the microstructure patch (91) to exit from said second processing area (124),
said coating device (3) is disposed in said coating area (123) to perform a coating process that imparts a transdermally active substance on the microstructure patch (91);
said processing unit includes a UV sterilization unit (61) disposed in said first processing area (122) to sterilize the microstructure patches (91), and a drying unit (62) disposed in said second processing area (124) to dry the microstructure patches (91); and
said integrated microstructure patch processing device further comprises a control unit (8) electrically connected to said moving structure (22) of said support unit (2), said coating device (3), and said processing unit (6) to control operations of said moving structure of said support unit (2), said coating device (3), and said processing unit (6).

20. The integrated microstructure patch processing device as claimed in claim 19, further comprising a packaging device (7) disposed in said accommodation space (12) to cover and seal the microstructure patch (91) that arrives at said second processing area (124) with a packaging member (10), wherein:
said first processing area (122) and said second processing area (124) are aligned along a y-axis direction perpendicular to the rotation axis (L); and
said packaging device (7) has a suction unit (73) that is movable between the first processing area (122) and said second processing area (124) to pick up and transfer a packaging member (10) from said first processing area (122) to said second processing area (124) and that is operable to be placed on and to cover said packaging member (10) on the microstructure patch (91) arriving at said second processing area (122).
